# EUROPÄISCHE PATENTSCHRIFT

(11) **EP 4 241 824 B1**
(45) Veröffentlichungstag und Bekanntmachung des Hinweises auf die Patenterteilung: **12.02.2025**
(21) Anmeldenummer: 22160485.3
(22) Anmeldetag: 07.03.2022
(51) Int. Cl.: A61M 37/00

(54) **HANDGERÄT ZUM LOKALEN AUFSTECHEN EINER HAUT**
HAND TOOL FOR LOCALLY PIERCING A SKIN
DISPOSITIF À MAIN PERMETTANT D'EFFECTUER LOCALEMENT UNE PONCTION D'UNE PEAU

(43) Veröffentlichungstag der Anmeldung: 13.09.2023
(73) Patentinhaber: MT.DERM GmbH, 12307 Berlin (DE)
(72) Erfinder: SCHERKOWSKI, Dirk, 12489 Berlin (DE)
(74) Vertreter: Bittner, Thomas L.

(56) Entgegenhaltungen:
- DE-A1- 10 343 590
- DE-A1- 102005 058 767
- US-A- 464 801
- US-A1- 2013 123 825
- US-A1- 2018 369 553
- US-B1- 8 522 647

## Beschreibung

Die Erfindung betrifft ein Handgerät zum lokalen Aufstechen einer Haut.

### Hintergrund

Ein solches Handgerät wird verwendet, um eine menschliche oder tierische Haut lokal aufzustechen, zum Beispiel zum Herstellen einer Tätowierung oder für medizinische oder kosmetische Anwendungen. Beispielsweise kann das lokale Aufstechen der Haut dazu dienen, einen Farbstoff in oberen Hautschichten einzubringen, zum Beispiel zum Ausbilden einer Tätowierung oder von permanentem Makeup. Aber auch das Einbringen von medizinischen oder kosmetischen Stoffen kann vorgesehen sein.

Übliche Handgeräte weisen ein Gehäuse auf, in dem eine Antriebseinrichtung angeordnet ist, beispielsweise ein elektrischer Motor, welche im Betrieb eine maschinell erzeugte Antriebskraft bereitstellt, die über eine Kopplungseinrichtung auf eine Stecheinrichtung in dem Gehäuse übertragen wird, so dass die Stecheinrichtung vor- und zurückbewegt wird, wobei das Aufstechen der Haut regelmäßig am Ende der Vorwärtsbewegung stattfindet und die hierfür verwendeten Stechnadeln der Stecheinrichtung mittels der Zurückbewegung wieder aus der Haut herausgezogen werden. Zumindest in der ausgefahrenen Stellung sind hierbei Nadelspitzen der Stechnadel(n) außerhalb einer vorderen Gehäuseöffnung des Gehäuses angeordnet, so dass sie in die Haut eingestochen werden können. Die Einstechtiefe in die Haut hängt hierbei unter anderem davon ab, mit welcher Länge die Stechnadeln gegenüber der vorderen Gehäuseöffnung überstehen und wie groß ein Arbeitshub ist, den die Stechnadeln beim Verlagern in die ausgefahrene (vordere) Stellung ausführen.

US 2018/0369553 A1 betrifft eine Tätowiermaschine mit lösbaren, sterilisierbaren Teilen.

US 8,522,647 B1 betrifft ein Exzentergetriebe für eine Tätowiermaschine, wobei die Wurftiefe der Nadel durch den Verwender mittels des Exzentergetriebes einstellbar ist.

US 2018/0369553 A1 betrifft eine stiftartige Microneedling-Vorrichtung.

DE 103 43 590 A1 ein Aufsatzmodul für Tattoo- und Permanent-Make-up-(PMU-)Geräte mit einer Schutzhülle für den Einmalgebrauch.

DE 10 2005 058 767 A1 bezieht sich auf ein Hygienemodul, welches bei der Tattoo- bzw. Permanent-Make-up-Erstellung am menschlichen Körper eingesetzt wird und hierbei die Farbzuführung reguliert bzw. minimal Partikel durch die Hubbewegung des Nadelsystems erzeugt.

US 2013/0123825 A1 betrifft eine Vorrichtung zum Einstechen in organisches Gewebe, insbesondere menschliche oder tierische Haut.

### Zusammenfassung

Aufgabe der Erfindung ist es, ein Handgerät zum lokalen Aufstechen einer Haut mit verbesserten Betriebseigenschaften anzugeben.

Zur Lösung ist ein Handgerät zum lokalen Aufstechen einer Haut nach Anspruch 1 geschaffen. Ausgestaltungen sind Gegenstand von abhängigen Ansprüchen.

Nach einem Aspekt ist ein Handgerät zum lokalen Aufstechen einer Haut geschaffen, welches Folgendes aufweist: ein Gehäuse; eine Antriebseinrichtung, die in dem Gehäuse angeordnet und eingerichtet ist, eine Antriebskraft bereitzustellen; eine Stecheinrichtung, die in dem Gehäuse angeordnet ist und mindestens eine an einem Nadelträger angeordnete Stechnadel aufweist; und eine Kopplungseinrichtung, die in dem Gehäuse angeordnet ist und die Antriebseinrichtung mit der Stecheinrichtung verbindet, derart, dass die Antriebskraft auf die Stecheinrichtung übertragbar ist, um den Nadelträger mit der mindestens einen Stechnadel im Betrieb wiederholt zwischen einer vorderen und einer hinteren Stellung zu bewegen. Die Kopplungseinrichtung weist eine Hebelmechanik auf, die eingerichtet ist, beim Übertragen der Antriebskraft antriebsseitig über einen Eingang der Hebelmechanik einen von der Antriebseinrichtung bereitgestellten Antriebshub zu empfangen, den Antriebshub in einen Arbeitshub zu wandeln, welcher von dem Antriebshub verschieden ist, und den Arbeitshub über einen Ausgang der Hebelmechanik zur Stecheinrichtung hin abzugeben.

Die vorgesehene Hebelmechanik in der Kopplungseinrichtung ermöglicht es, zum Übertragen der Antriebskraft von der Antriebseinrichtung auf die Stecheinrichtung den antriebsseitig bereitgestellten Antriebshub gezielt zu verändern, indem die Hebelmechanik den Antriebshub in den Arbeitshub wandelt, welcher von dem Antriebshub verschieden ist. Hierdurch kann für den Betrieb der Stecheinrichtung ein Arbeitshub bereitgestellt werden, welcher nicht auf den antriebsseitig bereitgestellten Antriebshub beschränkt ist. Je nach gewünschter Ausführung des Handgeräts kann der antriebsseitig bereitgestellte Antriebshub anwendungsbezogen verändert und in den Arbeitshub gewandelt werden.

Die Hebelmechanik kann eingerichtet sein, den Antriebshub mit einer Antriebshublänge in den Arbeitshub mit einer Arbeitshublänge zu wandeln, die von der Antriebshublänge verschieden ist. Bei dieser Ausführungsform ist vorgesehen, dass der Antriebshub mit Hilfe der Hebelmechanik verlängert wird, was beispielsweise die Bereitstellung eines größeren Nadel-überstands der Stechnadelspitzen gegenüber der vorderen Gehäuseöffnung ermöglicht, hierdurch wahlweise eine größere Einstechtiefe im Betrieb des Handgeräts beim lokalen Aufstechen der Haut.

Die Arbeitshublänge kann größer sein als die Antriebshublänge. Alternativ kann die Arbeitshublänge kleiner sein als die Antriebshublänge. Hierdurch ist es ermöglicht, eine Antriebseinrichtung auch in Handgeräten zu verwenden, die im Betrieb einen Arbeitshub bereitstellen sollen, der kürzer oder kleiner ist als der von der Antriebseinrichtung bereitgestellte Antriebshub.

In verschiedenen Anwendungen kann vorgesehen sein, dass mit Hilfe der Hebelmechanik die Bewegungsrichtung des ursprünglichen Antriebshubs für den Arbeitshub umgekehrt oder invertiert wird.

Die Hebelmechanik kann mit mindestens zwei zusammenwirkenden Hebeln gebildet sein. Die mindestens zwei zusammenwirkenden Hebel können an einem Lagerbauteil schwenkbar gelagert sein, beispielsweise in einer jeweils zugeordneten Lagerung, wobei der jeweilige Lagerung Begrenzungsmittel zugeordnet sind, welche eine Schwenk- oder Drehbewegung des jeweiligen Hebels im Betrieb der Hebelmechanik bei der Hubwandlung begrenzen.

Die Hebelmechanik kann zwischen dem Eingang und dem Ausgang als eine starre Hebelmechanik ausgeführt sein. Eine starre Hebelmechanik bedeutet insbesondere, dass Elemente oder Bauteile der Hebelmechanik, zum Beispiel die mindestens zwei zusammenwirkenden Hebel, fest miteinander in Verbindung stehen, beispielsweise ein Abstand zwischen der jeweiligen Lagerung von Hebeln der Hebelmechanik im Betrieb gleichbleibend fixiert ist. Aufgrund der mittels der starren Hebelmechanik ausgebildeten festen Verbindung kann eine (Kraft-)Übertragungscharakteristik, die mittels der Hebelmechanik zwischen dem Eingang und dem Ausgang ausgebildet ist, während des Betriebs zum Vor- und Zurückbewegen des Nadelträgers mit der mindestens einen Stechnadel jederzeit konstant sein.

Der Nadelträger mit der mindestens einen Stechnadel kann beim Verlagern aus der hinteren in die vordere Stellung gegen eine von einer Rückstelleinrichtung bereitgestellten Rückstellkraft verlagerbar sein, und die Rückstellkraft kann eingerichtet sein, eine Gesamtrückstellkraft, die für eine Verlagerung des Nadelträgers mit der mindestens einen Stechnadel aus der vorderen Stellung in die hintere Stellung aufzubringen ist, zumindest teilweise bereitzustellen. Der Nadelträger mit der mindestens einen Stechnadel wird nach dem Verlagern in die vordere Stellung, bei der es sich in Bezug zur vorderen Gehäuseöffnung für die Stechnadelspitze(n) um eine ausgefahrene Stellung handelt, mit Hilfe der Gesamtrückstellkraft in die hintere Stellung zurückverlagert.

Diese Verlagerung zwischen der vorderen und der hinteren Stellung wird im Betrieb wiederholt mit einer Wiederholfrequenz ausgeführt, so dass das Handgerät benutzt werden kann, um eine Haut lokal mehrfach aufzustechen. Die wahlweise von der Rückstelleinrichtung bereitgestellte Rückstellkraft kann in einem Ausführungsbeispiel der Gesamtrückstellkraft vollständig entsprechen. Die Rückstelleinrichtung kann ein oder mehrere Rückstellelemente aufweisen, die zum Beispiel von einem Federelement oder mehreren Federelementen gebildet sind. Hierbei kann vorgesehen sein, dass die Federelemente beim Verlagern des Nadelträgers mit der mindestens einen Stechnadel in die vordere Stellung gestreckt werden und so die Rückstellkraft entwickeln, die dann zum Zurückbewegen des Nadelträgers (Zurückziehen) auf diesen oder ein anderes Element der bewegten Teile, beispielsweise der Stecheinrichtung, eingekoppelt wird. Zum Ausbilden einer anteilige Rückstellkraft oder einer Gesamt-Rückstellkraft kann beispielsweise auch der Hebelmechanik eingerichtet sein, insbesondere basierend auf der Wirkung eines Drehmoments um den Drehpunkt eines oder mehrerer Hebel.

Alternativ zum Vorsehen einer Rückstelleinrichtung kann das Handgerät frei von einer solchen (wahlweise ergänzenden) Rückstelleinrichtung ausgeführt sein, derart, dass unter Einbeziehung der Hebelmechanik eine (insbesondere lösbare) starre (kraftschlüssige) Kopplung zwischen Antriebseinrichtung und Stecheinrichtung ausgebildet ist, so dass - neben der Antriebskraft zum Verlagern in die vordere Stellung (Druckkraft) - auch die Rückstellkraft zum Verlagern aus der vorderen in die hintere Stellung direkt von der Antriebseinrichtung bereitgestellt ist und über die starre (kraftschlüssige) Verbindung auf die Stecheinrichtung übertragen wird (Zugkraft).

In dem Gehäuse kann eine Dichtmembran einen vorderen Gehäuseraum von einem hinteren Gehäuseraum dichtend voneinander trennen. Der vordere Gehäuseraum kann sich zu der vorderen Gehäuseöffnung hin öffnen. Die Dichtmembran verhindert insbesondere, dass Körperflüssigkeit, die bei der Verwendung des Handgeräts zum lokalen Aufstechen der Haut durch die vordere Gehäuseöffnung in den vorderen Gehäuseraum gelangen kann, dann auch in den hinteren Gehäuseraum eindringt. Im hinteren Gehäuseraum kann die Antriebseinrichtung zumindest teilweise aufgenommen sein. Im vorderen Gehäuseraum kann die Stecheinrichtung zumindest teilweise angeordnet sein, insbesondere der Nadelträger mit der mindestens einen Stechnadel.

Die Rückstelleinrichtung kann mit der Dichtmembran gebildet sein, derart, dass die Rückstellkraft zumindest teilweise ausgebildet ist, wenn ein oder mehrere elastische Membranabschnitte der Dichtmembran beim Verlagern des Nadelträgers mit der mindestens einen Stechnadel aus der hinteren in die vordere Stellung getreckt werden. Wird der Nadelträger mit der mindestens einen Stechnadel im Betrieb in die vordere Stellung verlagert, so strecken sich die elastischen Membranabschnitte der Dichtmembran, so dass die Rückstell- oder Rückholkraft von der Dichtmembran selbst bereitgestellt ist. Ein Kopplungsabschnitt der Dichtmembran ist hierbei fest, insbesondere nicht rutschend, mit dem Nadelträger verbunden, sei es direkt oder indirekt über ein Zwischenbauteil, so dass die elastischen Membranabschnitte beim Verlagern in die vordere Stellung gestreckt werden und dann die Dichtmembran ihre Rückstellkraft auf den Nadelträger einkoppeln kann, um dessen Verlagerung aus der vorderen zu der hinteren Stellung hin zu unterstützen oder allein zu bewirken.

Die Hebelmechanik kann in dem hinteren Gehäuseraum angeordnet sein. Auf diese Weise ist die Hebelmechanik mittels der Dichtmembran gegenüber dem vorderen Gehäuseraum abgedichtet. Die Hebelmechanik kann zumindest teilweise in dem vorderen Gehäuseraum angeordnet sein.

Der Nadelträger der Stecheinrichtung kann direkt mit dem Ausgang der Hebelmechanik verbunden sein. Alternativ kann eine indirekte Verbindung oder Kopplung zwischen dem Nadelträger und der Hebelmechanik über ein dem Nadelträger zugeordnetes Zwischenbauteil ausgebildet sein, beispielsweise über ein oder mehrere Verbindungsbauteile.

Ein Ausgangselement, an dem der Ausgang der Hebelmechanik gebildet ist, kann in einer zugeordneten Aufnahme an dem Nadelträger der Stecheinrichtung schwenkbar gelagert sein. Beispielsweise kann ein im Wesentlichen quer zur Längsrichtung des Gehäuses verlaufender Hebelabschnitt eines der Hebel der Hebelmechanik in der zugeordneten Aufnahme an dem Nadelträger schwenkbar aufgenommen sein.

Das Gehäuse kann mehrstückig mit voneinander lösbaren Gehäuseteilen ausführt sein. Das Gehäuse ist hierbei mehrteilig mit mehreren Gehäuseteilen ausgeführt, die voneinander lösbar sind. Die Gehäuseteile können Einweg- und / oder Mehrwegmodule umfassen. Einwegmodule sind für eine Einmalnutzung ausgebildet und werden nach der Nutzung des Handgeräts zum Hautaufstechen ausgetauscht. Mehrwegmodule werden hingegen mehrfach verwendet.

Die voneinander lösbaren Gehäuseteile können ein oder mehrere der funktionellen Komponenten oder Einrichtungen des Handgeräts jeweils aufnehmen. Weisen zwei Gehäuseteile jeweils eine funktionelle Komponente oder Einrichtung des Handgeräts auf, können Kopplungsmittel vorgesehen sein, die die beiden funktionellen Komponenten beim Verbinden der Gehäuseteile koppeln sowie entkoppeln, wenn die Gehäuseteile voneinander getrennt oder gelöst werden. Das kann beispielsweise ein Kopplungsmittel zum Verbinden der Antriebseinrichtung in einem Gehäuseteil mit einer die Antriebsbewegung bzw. die Antriebskraft aufnehmenden funktionellen Komponente in einem anderen Gehäuseteil betreffen. Die Kopplungsmittel können beispielsweise eine Steck-, Klemm- und / oder Schraubverbindung zum Koppeln aufweisen.

Mit einem hinteren Gehäuseteil, in welchem die Antriebseinrichtung angeordnet ist, kann ein Handstück und mit einem vorderen Gehäuseteil kann ein Hautstechwechselmodul gebildet sein, in welchem die Stecheinrichtung angeordnet ist. Das Handstück wird vom Nutzer im Betrieb des Handgeräts mit den Fingern gegriffen, um das Handgerät zu führen und kann als ein Mehrwegmodul ausgebildet sein. Das hintere Gehäuseteil kann seinerseits ein oder mehrere Teilgehäuseelemente umfassen. Das Hautstechwechselmodul mit der Stecheinrichtung kann als Einwegmodul ausgebildet sein, so dass es an einem anderen Gehäuseteil, insbesondere dem hinteren Gehäuseteil, wechselbar angebracht ist. In einer Ausgestaltung ist ein Gehäuseinnenraum des Hautstechwechselmoduls Teil des vorderen Gehäuseraums, welcher mittels der Dichtmembran gegenüber dem hinteren Gehäuseraum dichtend abgetrennt ist.

Die Hebelmechanik kann zumindest teilweise in dem Hautstechwechselmodul angeordnet sein.

Das hintere Gehäuseteil kann ein Handstück mit Antriebseinrichtung einer elektrischen Zahnbürste sein. Das Handstück umfasst die Antriebsreinrichtung und ist eingerichtet, an einen Aufsatz mit einer Bürsteneinrichtung lösbar zu koppeln, so dass - in Ergänzung zur Hautstecheinrichtung - eine elektrische Zahnbürste bereitgestellt ist. Auf diese Weise kann ein Artikel bereitgestellt sein, welcher das Handstück mit der Antriebseinrichtung einer elektrischen Zahnbürste sowie ein Hautstechwechselmodul und ein Bürsten(wechsel)modul mit der Bürsteneinrichtung umfasst, die jeweils an das Handstück mit der Antriebseinrichtung lösbar gekoppelt werden können, derart, dass die von der Antriebseinrichtung bereitgestellte Antriebskraft oder -bewegung auf das jeweils angekoppelte Modul eingekoppelt werden kann.

Die Hebelmechanik kann in dem vorderen Gehäuseteil angeordnet sein. Die Hebelmechanik kann hierbei in dem Hautstechwechselmodul angeordnet sein. Alternativ kann die Hebelmechanik in einem Kopplungsmodul angeordnet sein, welches lösbar mit dem Handstück der elektrischen Zahnbürste verbindbar ist. Die Stecheinrichtung kann dann in dem Hautstechwechselmodul angeordnet sein, welches an dem Kopplungsmodul lösbar angeordnet ist.

Das Handgerät kann einen Einstellmechanismus aufweisen, der eingerichtet ist, einen Nadelherausstand oder Nadelüberstand einzustellen, also einen Überstand oder Herausstand der Nadelspitzen in Bezug auf eine vordere Gehäusekante oder eine vordere Gehäuseöffnung, durch welche sich die Stechnadeln beim Verlagern in die vordere (ausgefahrene) Stellung bewegen. Bei dem Einstellmechanismus kann ein Gehäuseteil am vorderen Modulgehäuse relativ zum Nadelträger und somit zu den Stechnadeln verstellbar sein. Zum Beispiel kann das Gehäuseteil nach dem Koppeln des Hautstechwechselmoduls an das Handstück im Bereich eines hinteren Gehäuseabschnitts mit einer in Längsrichtung des Gehäuses verstellbaren oder verlagerbaren Hülse in Verbindung stehen (an diese koppeln), derart, dass ein Verstellen oder Verlagern der Hülse in Längsrichtung des Gehäuses, beispielweise mittels Drehen der Hülse entlang einer Gewindelagerung der Hülse am hinteren Gehäuseteil, die Relativlage des Gehäuseteils in Bezug auf den Nadelträger mit den Stechnadeln und somit in Bezug auf die Nadelspitzen ändert, so dass hierdurch ein Nadelüberstand oder Nadelherausstand der Nadelspitzen relativ zur vorderen Gehäusekante eingestellt wird.

In einer Ausführung ist eine Stechnadelspitze der mindestens einen Stechnadel, zumindest in der vorderen Stellung, über eine vordere Gehäuseöffnung überstehend angeordnet. Alternativ kann die Stechnadelspitze sowohl in der vorderen wie auch in der hinteren Stellung hinter der vorderen Gehäuseöffnung angeordnet sein, wobei die Stechnadelspitze in der vorderen Stellung näher zur vorderen Gehäuseöffnung angeordnet ist als in der hinteren Stellung, wahlweise im Bereich der vorderen Gehäuseöffnung.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische perspektivische Darstellung eines Handgeräts zum lokalen Aufstechen einer Haut;
- Fig. 2: eine schematische perspektivische Darstellung eines Hautstechwechselmoduls;
- Fig. 3: eine schematische perspektivische Darstellung des Hautstechwechselmoduls aus Fig. 2 im Schnitt;
- Fig. 4: eine weitere schematische perspektivische Darstellung des Hautstechwechselmoduls aus Fig. 2 im Schnitt;
- Fig. 5: eine schematische perspektivische Darstellung eines anderen Hautstechwechselmoduls;
- Fig. 6: eine schematische perspektivische Darstellung des anderen Hautstechwechselmoduls aus Fig. 5 teilweise im Schnitt;
- Fig. 7: eine weitere schematische perspektivische Darstellung des anderen Hautstechwechselmoduls aus Fig. 5, wobei Stechnadeln weiter eingefahren sind;
- Fig. 8: eine weitere schematische perspektivische Darstellung des anderen Hautstechwechselmoduls aus Fig. 5 im Schnitt;
- Fig. 9: eine schematische perspektivische Darstellung von Elementen des anderen Hautstechwechselmoduls in Fig. 5;
- Fig. 10: eine weitere schematische perspektivische Darstellung von Elementen des anderen Hautstechwechselmoduls aus Fig. 5 im Schnitt;
- Fig. 11: eine schematische perspektivische Darstellung des anderen Hautstechwechselmoduls aus Fig. 5, wobei die Stechnadeln in einer Zwischenstellung angeordnet sind;
- Fig. 12: eine schematische perspektivische Darstellung des anderen Hautstechwechselmoduls aus Fig. 5 im Schnitt, wobei die Stechnadeln in einer ausgefahrenen (vorderen) Stellung angeordnet sind;
- Fig. 13: eine schematische perspektivische Darstellung einer weiteren Ausführungsform eines Handgeräts zum lokalen Aufstechen einer Haut mit einem Handstück einer elektrischen Zahnbürste;
- Fig. 14: eine schematische perspektivische Teildarstellung des Handgeräts aus Fig. 13 und
- Fig. 15: eine schematische perspektivische Teildarstellung des Handgeräts aus Fig. 13.

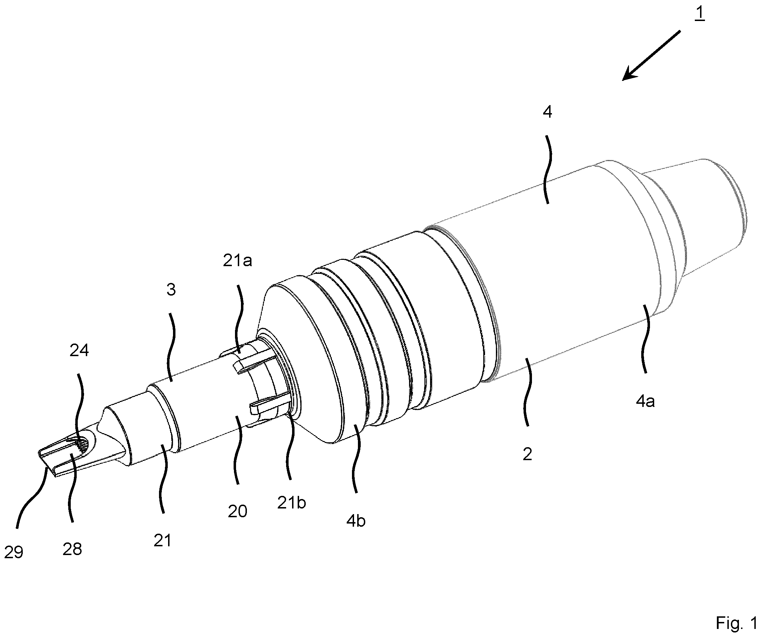

Fig. 1 zeigt eine schematische perspektivische Darstellung eins Ausführungsbeispiels für ein Handgerät 1 zum lokalen Aufstechen einer Haut. Das Handgerät 1 weist ein Gehäuse 2 mit einem vorderen und einem hinteren Gehäuseteil 3, 4 auf, die bei dem gezeigten Ausführungsbeispiel lösbar miteinander verbunden sind. Mit dem hinteren Gehäuseteil 4 ist ein Handstück 4a bereitgestellt, welches der Nutzer im Betrieb mit Fingern greift.

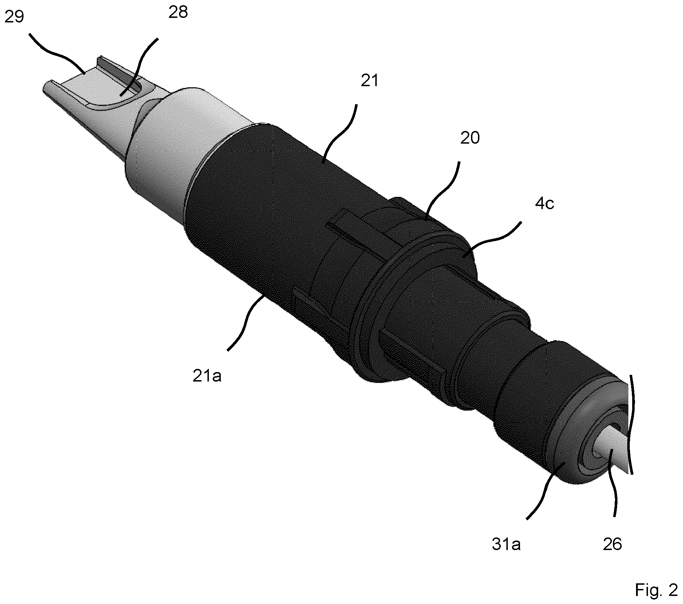

Die Fig. 2 bis 4 zeigen schematische perspektivische Darstellungen eines wechselbaren Hautstechmoduls (Hautstechwechselmodul) 20, welches bei dem Handgerät 1 aus Fig. 1 mit dem vorderen Gehäusebauteil 3 gebildet ist.

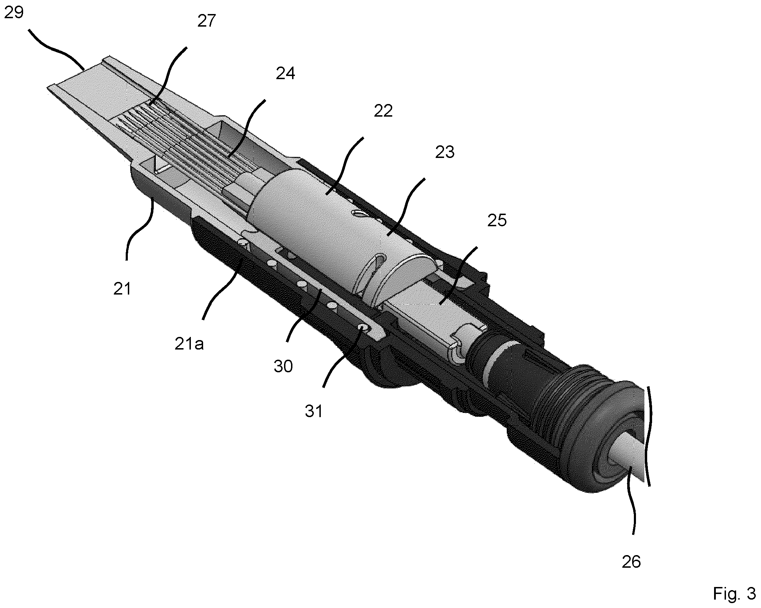

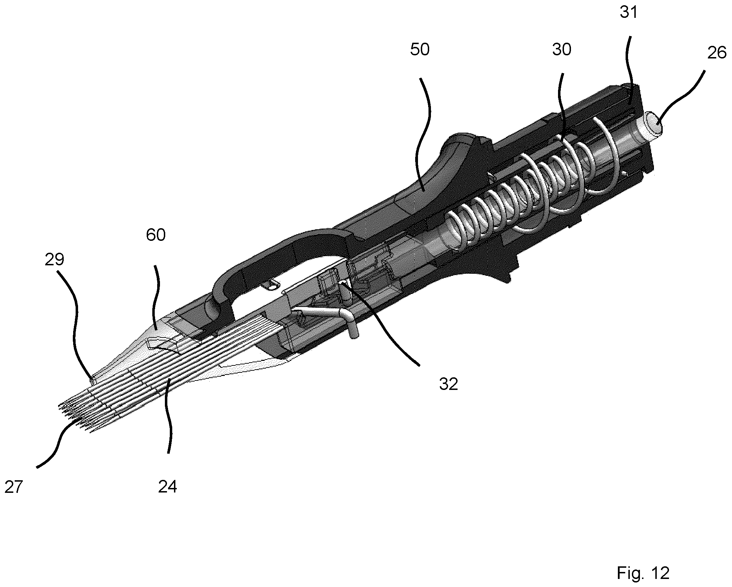

In einem Modulgehäuse 21 ist eine Stecheinrichtung 22 angeordnet, bei der an einem Nadelträger 23 Stechnadeln 24 angeordnet sind. Der Nadelträger 23 ist mit einer modulseitigen Kopplungseinrichtung 25 verbunden, derart, dass im Betrieb aufgrund eines über ein Kopplungsbauteil 26 bereitgestellte Antriebskraft, die von einer Antriebseinrichtung in dem Gehäuse 2 bereitgestellt wird, auf den Nadelträger 23 übertragen wird, so dass der Nadelträger 23 mit den hieran angeordneten Stechnadeln 24 in Gehäuselängsrichtung zwischen einer vorderen und einer hinteren Stellung vor- und zurückbewegt wird. Fig. 3 zeigt den Nadelträger 23 mit den Stechnadeln 24 in der hinteren Stellung, in welcher Nadelspitzen 27 der Stechnadeln 24 hinter einer vorderen Gehäuseöffnung 28 (vgl. Fig. 2) angeordnet sind (eingefahrene Stellung). Zum Aufstechen der Haut wird der Nadelträger 23 mit den Stechnadeln 24 in eine vordere Stellung verlagert (vgl. Fig. 12 unten), in welcher die Nadelspitzen 27 gegenüber der vorderen Gehäuseöffnung 28 überstehen, insbesondere in Bezug auf eine vordere Gehäusekante 29 vorstehen.

Der Nadelträger 23 wird bei der Vor- und Zurückbewegung im Betrieb in dem Modulgehäuse 21 geführt. Ein Federelement 30 sorgt dafür, dass eine Gehäusehülse 31 mit einer Kappe 31a nach dem Andocken und unabhängig von der Einstellung eines Nadelüberstandes der Stechnadelspitze 27 in Bezug auf die vordere Gehäuseöffnung 28 oder die vordere Gehäusekante 29 gegen einen Boden einer Modulaufnahme am Handstück 4a gepresst wird.

Ein Gehäuseteil 21a am Modulgehäuse 21 steht nach dem Koppeln des Hautstechmoduls 20 an das Handstück im Bereich eines hinteren Gehäuseabschnitts 21b mit einer in Längsrichtung des Gehäuses 2 verstellbaren oder verlagerbaren Hülse 4b (vgl. Fig. 1) in Verbindung, derart, dass ein Verstellen oder Verlagern der Hülse in Längsrichtung des Gehäuses 2, beispielweise mittels Drehen der Hülse 4b entlang einer Gewindelagerung der Hülse 4b am hinteren Gehäuseteil 4, die Relativlage des Gehäuseteils 21a in Bezug auf den Nadelträger 23 mit den Stechnadeln 24 und somit in Bezug auf die Nadelspitzen 27 ändert, so dass hierdurch ein Nadelüberstand oder Nadelherausstand der Nadelspitzen 27 relativ zur vorderen Gehäusekante 29 eingestellt wird.

Das Gehäuseteil 21a stößt an einen umlaufenden Vorsprung 21c am Modulgehäuse 21, welcher einen Anschlag für das Gehäuseteil 21a bildet.

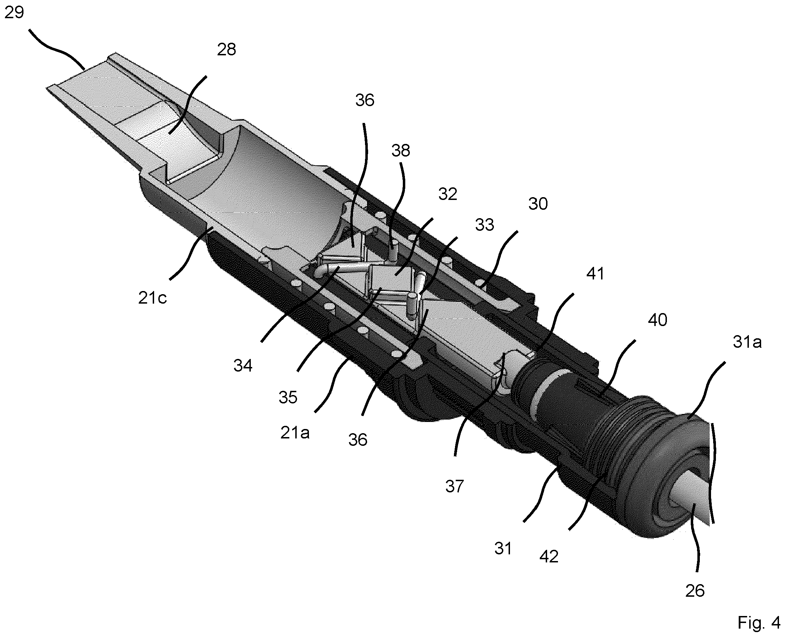

Das Verlagern in die vordere Stellung nach vorn zur vorderen Gehäuseöffnung 28 hin erfolgt bei dem gezeigten Ausführungsbeispiel gegen eine Rückstellkraft einer Dichtmembran 40, die hierbei gestreckt wird (vgl. Fig. 4). Die Dichtmembran 40 ist dann Teil einer Rückstelleinrichtung, welche eine Rückstellkraft bereitstellt, und dichtet gleichzeitig einen vorderen Gehäuseraum 41 (vorderseitig in Bezug auf die Dichtmembran 40 im Gehäuse des Handgeräts) gegen einen hinteren Gehäuseraum 42 (rückseitig in Bezug auf die Dichtmembran 40 im Gehäuse des Handgeräts, in Richtung der Antriebseinrichtung) ab. Auch in Bezug auf das Modulgehäuse 21 unterteilt die Dichtmembran 40 dieses in einen vorderen und einen hinteren Gehäuseraum innerhalb des Modulgehäuses 21.

Die Dichtmembran aus einem elastischen Material trägt dann auch zum Zurückbewegen des Nadelträgers 23 mit den Stechnadeln 24 in die hintere (eingefahrene) Stellung gemäß Fig. 3 bei, nachdem der Nadelträger 23 mit den Stechnadeln 24 zuvor in die vordere Stellung verlagert wurde. Auf diese Weise führt der Nadelträger 23 mit den Stechnadeln 24 im Betrieb, als Reaktion auf die eingekoppelte Antriebskraft, in Längsrichtung des Gehäuses 2 eine Vor- und Zurückbewegung mit einer Wiederholfrequenz aus, die zum Beispiel mit Hilfe eines elektrischen Motors der Antriebseinrichtung veränderbar sein kann.

Gemäß Fig. 4 ist in dem wechselbaren Hautstechmodel 20 eine Hebelmechanik 32 als Bestandteil eines modulseitigen Teils der Kopplungseinrichtung, mit der die Antriebskraft auf den Nadelträger 23 übertragen wird, vorgesehen, die bei dem gezeigten Ausführungsbeispiel mit einem ersten Hebel 33 und einem zweiten Hebel 34 gebildet ist, die an einem Lagerbauteil 35 jeweils schwenkbar gelagert sind. Das Lagerbauteil 35 weist Begrenzungsmittel 36 auf, die dem ersten und dem zweiten Hebel 33, 34 zugeordnet sind und deren Schwenkbewegung im Betrieb begrenzen. Mit Hilfe der Hebelmechanik 32 wird ein Antriebshub, welcher über das Kopplungsbauteil 26 auf einen Eingang 37 der Hebelmechanik 32 übertragen wird, gewandelt in einen Arbeitshub, der von der Hebelmechanik über einen Ausgang 38 an den Nadelträger 23 mit den Stechnadeln 24 übertragen wird, wobei der Arbeitshub von dem auf die Hebelmechanik 32 eingekoppelten Antriebshub verschieden ist, beispielsweise eine vergrößerte oder verkleinerte Hublänge aufweist. Auf diese Weise ist es ermöglicht, den von der Antriebseinrichtung empfangenen Antriebshub umzuwandeln oder umzusetzen in einen konkret auf den Nadelträger 23 mit den Stechnadeln 24 übertragenen Arbeitshub, welcher dann bestimmt, welchen Weg (Hub) die Stechnadeln 24, insbesondere deren Nadelspitzen 27 im Betrieb ausführen. Beispielsweise kann durch die Hubumsetzung auf den Überstand der Stechnadelspitzen 27 gegenüber der vorderen Gehäusekante 29 Einfluss genommen werden, und somit auf die Einstechtiefe beim lokalen Aufstechen der Haut mit Hilfe des Handgeräts 1.

Bei dem dargestellten Beispiel für die Hebelmechanik 32 ist diese hubvergrößernd ausgeführt, was bedeutet, dass der ausgekoppelte Arbeitshub größer als der eingekoppelte Antriebshub ist. Die Hublänge wird vergrößert. Bei alternativen Ausgestaltungen kann die Hublänge gleich bleiben oder mittels der Hebelmechanik 32 verkürzt werden.

Die von der Dichtmembran 40 bereitgestellte Rückstellkraft ergänzt beim gezeigten Ausführungsbeispiel optional die Hebelmechanik 32 zumindest beim Zurückbewegen der Stecheinrichtung.

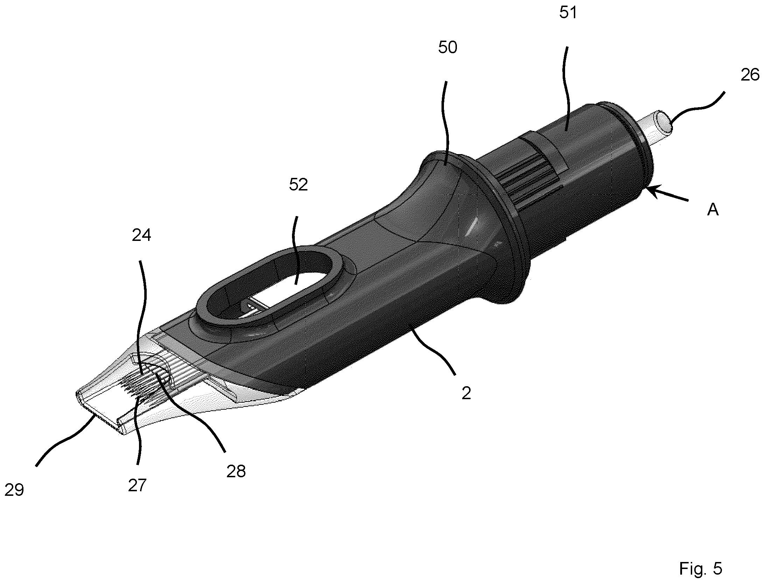

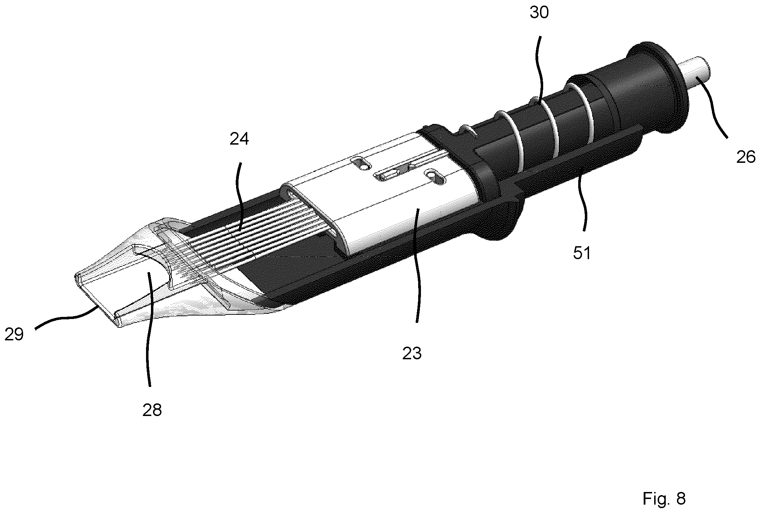

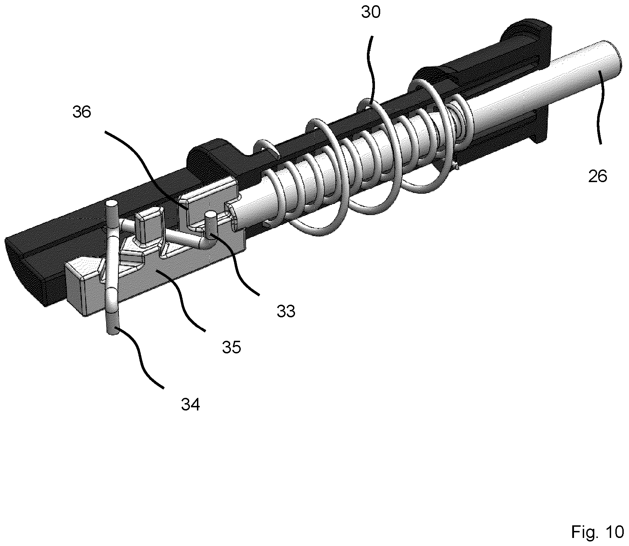

Fig. 5 bis 12 zeigen schematische Darstellungen für eine Ausführungsform eines anderen Nadelstechmoduls 50. Für gleiche Merkmale werden in den Fig. 5 bis 12 dieselben Bezugszeichen wie in den Fig. 2 bis 4 verwendet.

In den Fig. 5 bis 12 ist ein jeweils eingestellter Nadelheraus- oder Nadelüberstand, welchen die Nadelspitzen 27 in Bezug auf die vordere Gehäusekante 29 einnehmen, wenn die Stecheinrichtung in die vordere Stellung verlagert wird (vgl. Fig. 12), anhand eines jeweils eingestellten Abstandes A erkennbar (vgl. zum Beispiel Fig. 5 bis 7).

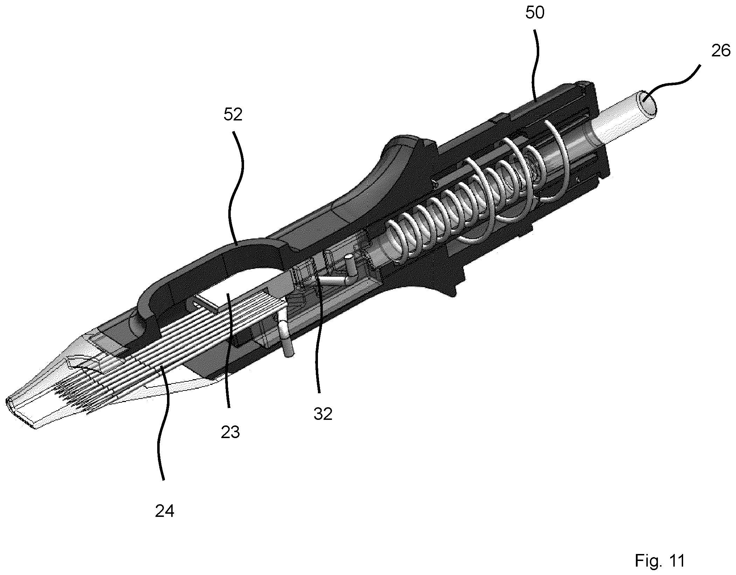

Hierzu ist ein Gehäuseteil 51 nach dem Koppeln an das Handstück 4a mittels der daran gebildeten Hülse 4b (vgl. Fig. 1) relativ zum Nadelträger 23 mit den Stechnadeln 24 und somit in Bezug auf die Nadelspitzen 27 einstellbar oder verlagerbar. Hierbei zeigt Fig. 5 (wie auch Fig. 12) für das andere Nadelstechmodul 50 eine Stellung der Nadelspitzen 27 mit einem eingestellten maximalen Nadelheraus- oder Nadelüberstand (Abstand A minimal). Fig. 11 zeigt eine Schnittdarstellung des anderen Nadelstechmoduls 50 in der in Fig. 5 gezeigten Stellung.

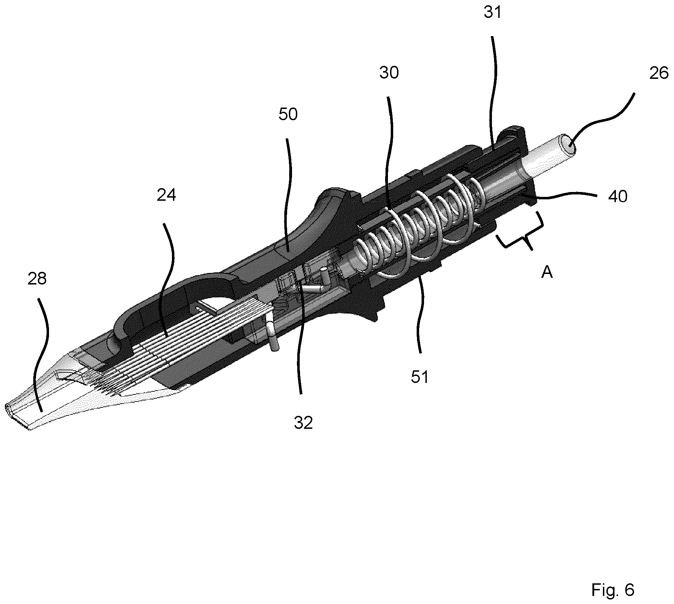

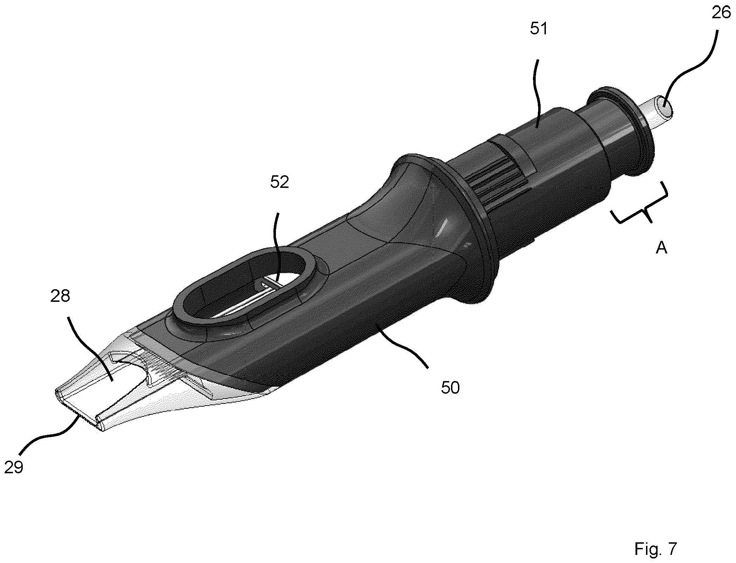

Fig. 7 zeigt einen minimalen Nadelheraus- oder Nadelüberstand, bei dem die Stechnadeln 24 zurückgezogen in der hinteren Stellung angeordnet sind. Die Fig. 6, 8 bis 10 zeigen unterschiedliche Schnittbilder des anderen Nadelstechmoduls 50 mit der in Fig. 7 gezeigten Stellung.

Wird das andere Nadelstechmodul 50 für einen Eintrag einer Substanz beim lokalen Aufstechen der Haut verwendet, so besteht die Möglichkeit, über eine Modulöffnung 52 die einzutragende Substanz einzubringen, so dass diese insbesondere aufgrund von Kapillarkräften entlang der Stechnadeln 24 zur aufgestochenen Haut hingelangen kann. Bei der Ausführungsform des Nadelstechmoduls 20 in den Fig. 2 bis 4 kann die Substanz, beispielsweise ein Farbstoff oder ein kosmetischer / medizinischer Wirkstoff im Bereich der vorderen Gehäuseöffnung 28 aufgebracht werden.

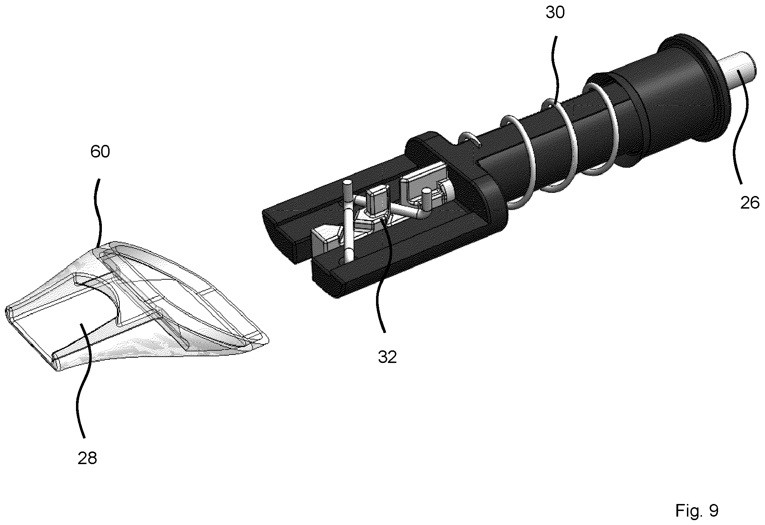

Gemäß Fig. 9 ist eine Modulspitze 60 lösbar an dem Modulgehäuse 21 angeordnet. Die Modulspitze 60 kann in Einheit mit den Stechnadeln 24 oder dem Nadelträger 23 (mit Stechnadeln 24) vom Modulgehäuse 21 lösbar angeordnet sein.

Während die Stechnadeln 24 insbesondere in den Fig. 6 bis 10 in einer hinteren (eingefahrenen) Stellung gezeigt sind, zeigt Fig. 5 eine Zwischenstellung, in welcher die Stechnadelspitzen 27 durch die vordere Gehäuseöffnung 28 einsehbar sind (vgl. auch Fig. 11). Fig. 12 zeigt schließlich die Stechnadeln 24 in einer vorderen (ausgefahrenen) Stellung (maximaler Nadelheraus- oder Nadelüberstand), in welcher die Stechnadelspitzen 27 gegenüber der vordere Gehäuseöffnung 28 wie auch der vorderen Gehäusekante 29 überstehen. Hierdurch ist ein Nadelüberstand ausgebildet, welcher durch eine Nadellänge charakterisiert ist, mit der die Stechnadeln 24 gegenüber der vorderen Gehäusekante 29 überstehen.

Auch bei den Beispielen in den Fig. 5 bis 12 ist die Hebelmechanik 32 hubvergrößernd ausgeführt.

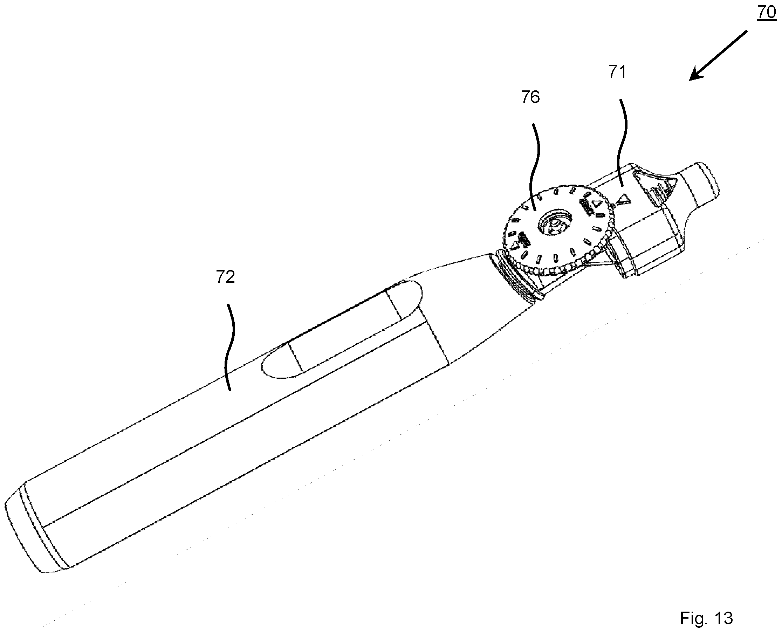

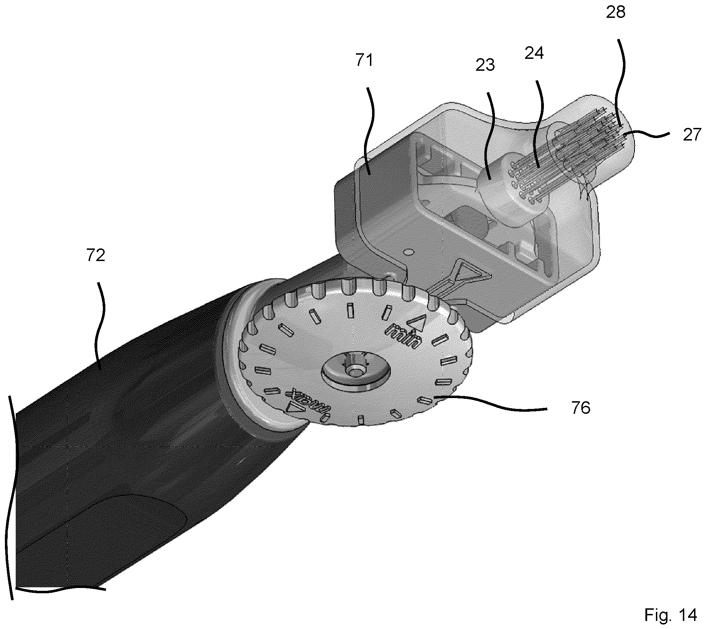

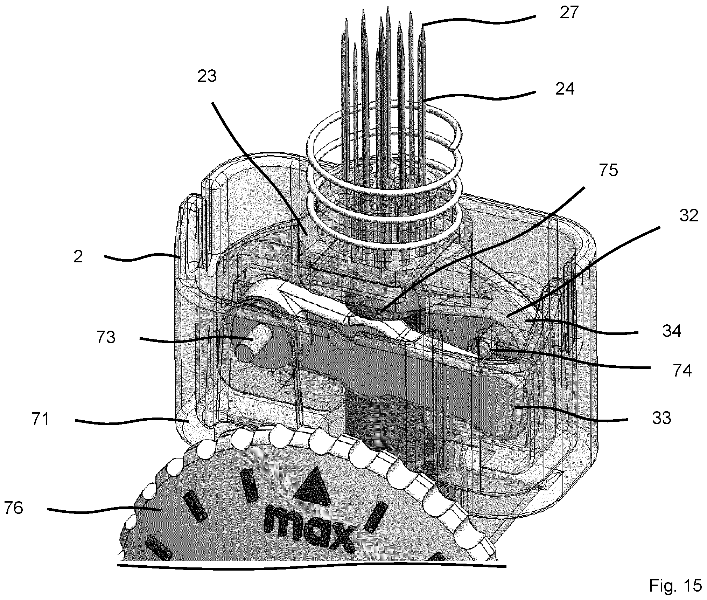

Fig. 13 zeigt eine schematische perspektivische Darstellung einer anderen Ausführungsform eines Handgeräts 70, bei dem ein aufgesetztes Nadelstechmodul 71 lösbar an einem Handstück 72 angeordnet ist, welches von einem Handstück einer elektrischen Zahnbürste mit hierin aufgenommener Antriebseinrichtung gebildet ist. Fig. 14 und 15 zeigen jeweils eine schematische perspektivische Teildarstellung des Handgeräts aus Fig. 13. Für gleiche Merkmale werden in den Fig. 13 bis 15 dieselben Bezugszeichen wie in den Fig. 1 bis 12 verwendet.

Die Hebelmechanik 32 ist mit dem ersten und dem zweiten Hebel 33, 34 gebildet, die in einer gekreuzten Anordnung gebildet sind. Der erste und der zweite Hebel 33, 34 sind mittels einer jeweiligen Lagerung 73, 74 am Modulgehäuse schwenkbar gelagert. Die Einleitung des Antriebshubs erfolgt über ein Koppelbauteil 75 auf die Hebelmechanik 32. Der gewandelte Arbeitshub wird dann an den Nadelträger 23 übertragen. Auf diese Weise wird die von dem Handstück der elektrischen Zahnbürste bereitgestellte Antriebskraft auf den Nadelträger 23 mit den Stechnadeln 24 übertragen, wobei hierbei die Hubumwandlung oder -umsetzung erfolgt.

Ein Einstellelement 76, welches im gezeigten Beispiel mit einem Drehelement gebildet ist, ist eingerichtet, einen Nadelüberstand oder Nadelherausstand für die Stechnadeln 24 gegenüber der vorderen Gehäuseöffnung 28 mittels Drehen des Drehelements einzustellen. Hierfür ist vorgesehen, dass das Drehelement mit einem Excenter-Mechanismus (nicht dargestellt) zusammenwirkt, derart, dass eine Stellung eines Hülsenbauteils 77, an dem die vordere Gehäuseöffnung 28 gebildet ist, relativ zu den Stechnadelspitzen 27 in der ausgefahrenen (vorderen) Stellung der Stechnadeln 24 veränderbar ist.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Handgerät zum lokalen Aufstechen einer Haut, mit
- einem Gehäuse (2);
- einer Antriebseinrichtung, die in dem Gehäuse (2) angeordnet und eingerichtet ist, eine Antriebskraft bereitzustellen;
- einer Stecheinrichtung (22), die in dem Gehäuse (2) angeordnet ist und mindestens eine an einem Nadelträger (23) angeordnete Stechnadel (24) aufweist; und
- einer Kopplungseinrichtung, die in dem Gehäuse (2) angeordnet ist und die Antriebseinrichtung mit der Stecheinrichtung (22) verbindet, derart, dass die Antriebskraft auf die Stecheinrichtung (22) übertragbar ist, um den Nadelträger (23) mit der mindestens einen Stechnadel (24) im Betrieb wiederholt zwischen einer vorderen und einer hinteren Stellung zu bewegen;
wobei die Kopplungseinrichtung eine Hebelmechanik (32) aufweist, die eingerichtet ist, beim Übertragen der Antriebskraft antriebsseitig über einen Eingang der Hebelmechanik (32) einen von der Antriebseinrichtung bereitgestellten Antriebshub zu empfangen, den Antriebshub in einen Arbeitshub zu wandeln, welcher von dem Antriebshub verschieden ist, und den Arbeitshub über einen Ausgang der Hebelmechanik (32) zur Stecheinrichtung (22) hin abzugeben, wobei das Gehäuse (2) mehrstückig mit voneinander lösbaren Gehäuseteilen ausgeführt ist, wobei mit einem hinteren Gehäuseteile, in welchem die Antriebseinrichtung angeordnet ist, ein Handstück und mit einem vorderen Gehäuseteil ein Hautstechwechselmodul gebildet sind, in welchem die Stecheinrichtung (22) angeordnet ist, **dadurch gekennzeichnet, dass** das hintere Gehäuseteil ein Handstück mit Antriebseinrichtung einer elektrischen Zahnbürste ist.

2. Handgerät nach Anspruch 1, **dadurch gekennzeichnet, dass** die Hebelmechanik (32) eingerichtet ist, den Antriebshub mit einer Antriebshublänge in den Arbeitshub mit einer Arbeitshublänge zu wandeln, die von der Antriebshublänge verschieden ist.

3. Handgerät nach Anspruch 2, **dadurch gekennzeichnet, dass** die Arbeitshublänge größer ist als die Antriebshublänge.

4. Handgerät nach Anspruch 2, **dadurch gekennzeichnet, dass** die Arbeitshublänge kleiner ist als die Antriebshublänge.

5. Handgerät nach mindestens einem der vorangehenden Ansprüche, **dadurch gekennzeichnet, dass** die Hebelmechanik (32) mit mindestens zwei zusammenwirkenden Hebeln (33, 34) gebildet ist.

6. Handgerät nach mindestens einem der vorangehenden Ansprüche, **dadurch gekennzeichnet, dass** die Hebelmechanik (32) zwischen dem Eingang und dem Ausgang als eine starre Hebelmechanik (32) ausgeführt ist.

7. Handgerät nach mindestens einem der vorangehenden Ansprüche, **dadurch gekennzeichnet, dass** der Nadelträger (23) mit der mindestens einen Stechnadel (24) beim Verlagern aus der hinteren in die vordere Stellung gegen eine von einer Rückstelleinrichtung bereitgestellten Rückstellkraft verlagerbar ist und die Rückstellkraft eingerichtet ist, eine Gesamtrückstellkraft, die für eine Verlagerung des Nadelträgers (23) mit der mindestens einen Stechnadel (24) aus der vorderen Stellung in die hintere Stellung aufzubringen ist, zumindest teilweise bereitzustellen.

8. Handgerät nach mindestens einem der vorangehenden Ansprüche, **dadurch gekennzeichnet, dass** in dem Gehäuse (2) eine Dichtmembran einen vorderen Gehäuseraum von einem hinteren Gehäuseraum dichtend voneinander trennt.

9. Handgerät nach Anspruch 8, **dadurch gekennzeichnet, dass** die Rückstelleinrichtung mit der Dichtmembran gebildet ist, derart, dass die Rückstellkraft ausgebildet ist, wenn elastische Membranabschnitte der Dichtmembran beim Verlagern des Nadelträgers (23) mit der mindestens einen Stechnadel (24) aus der hinteren in die vordere Stellung getreckt wird.

10. Handgerät nach Anspruch 8 oder 9, **dadurch gekennzeichnet, dass** die Hebelmechanik (32) in dem hinteren Gehäuseraum angeordnet ist.

11. Handgerät nach mindestens einem der vorangehenden Ansprüche, **dadurch gekennzeichnet, dass** der Nadelträger (23) der Stecheinrichtung (22) direkt mit dem Ausgang der Hebelmechanik (32) verbunden ist.

12. Handgerät nach Anspruch 11, **dadurch gekennzeichnet, dass** ein Ausgangselement, an dem der Ausgang der Hebelmechanik (32) gebildet ist, in einer zugeordneten Aufnahme an dem Nadelträger (23) der Stecheinrichtung (22) schwenkbar gelagert ist.

13. Handgerät nach Anspruch 1, **dadurch gekennzeichnet, dass** die Hebelmechanik in dem Hautstechwechselmodul angeordnet ist.

14. Handgerät nach Anspruch 1, **dadurch gekennzeichnet, dass** die Hebelmechanik in einem Kopplungsmodul angeordnet ist, das lösbar mit dem Handstück der elektrischen Zahnbürste verbindbar ist.

15. Handgerät nach mindestens einem der vorangehenden Ansprüche , soweit nicht auf Anspruch 10 rückbezogen, **dadurch gekennzeichnet, dass** die Hebelmechanik (32) in dem vorderen Gehäuseteil angeordnet ist.

## Claims

1. A hand-held device for locally piercing skin, with:
- a housing (2);
- a drive device, which is disposed in the housing (2) and is configured to provide a driving force;
- a piercing device (22), which is disposed in the housing (2) and has at least one piercing needle (24) disposed on a needle carrier (23); and
- a coupling device, which is disposed in the housing (2) and which connects the drive device to the piercing device (22) such that the driving force can be transmitted to the piercing device (22) in order to move the needle carrier (23) with the at least one piercing needle (24) repeatedly between a forward and a rearward position during operation;
wherein the coupling device has a lever mechanism (32) which is configured to receive a driving stroke provided by the drive device via an input of the lever mechanism (32) on the drive side when the driving force is transmitted, to convert the driving stroke into a working stroke which is different from the driving stroke, and to deliver the working stroke to the piercing device (22) via an output of the lever mechanism (32), wherein the housing (2) is constructed in multiple pieces with housing parts which can be detached from one another, wherein a handpiece is formed with a rear housing part in which the drive device is disposed, and an interchangeable skin piercing module is formed with a front housing part in which the piercing device (22) is disposed, **characterized in that** the rear housing part is a handpiece with a drive unit of an electric toothbrush.

2. The hand-held device as claimed in claim 1, **characterized in that** the lever mechanism (32) is configured to convert the driving stroke with a driving stroke length into the working stroke with a working stroke length which is different from the driving stroke length.

3. The hand-held device as claimed in claim 2, **characterized in that** the working stroke length is greater than the driving stroke length.

4. The hand-held device as claimed in claim 2, **characterized in that** the working stroke length is smaller than the driving stroke length.

5. The hand-held device as claimed in at least one of the preceding claims, **characterized in that** the lever mechanism (32) is formed with at least two interacting levers (33, 34).

6. The hand-held device as claimed in at least one of the preceding claims, **characterized in that** between the input and the output, the lever mechanism (32) is constructed as a rigid lever mechanism (32).

7. The hand-held device as claimed in at least one of the preceding claims, **characterized in that** during displacement from the rearward position into the forward position, the needle carrier (23) with the at least one piercing needle (24) is displaceable against a restoring force provided by a restoring device, and the restoring force is configured to at least partially provide a total restoring force which is to be applied for a displacement of the needle carrier (23) with the at least one piercing needle (24) from the forward position into the rearward position.

8. The hand-held device as claimed in at least one of the preceding claims, **characterized in that** a sealing membrane in the housing (2) separates a front housing chamber from a rear housing chamber in a sealing manner.

9. The hand-held device as claimed in claim 8, **characterized in that** the restoring device is formed with the sealing membrane such that the restoring force is developed when elastic membrane sections of the sealing membrane are stretched during the displacement of the needle carrier (23) with the at least one piercing needle (24) from the rearward position into the forward position.

10. The hand-held device as claimed in claim 8 or claim 9, **characterized in that** the lever mechanism (32) is disposed in the rear housing chamber.

11. The hand-held device as claimed in at least one of the preceding claims, **characterized in that** the needle carrier (23) of the piercing device (22) is directly connected to the output of the lever mechanism (32).

12. The hand-held device as claimed in claim 11, **characterized in that** an output element, at which the output of the lever mechanism (32) is formed, is pivotally mounted in an associated receptacle on the needle carrier (23) of the piercing device (22).

13. The hand-held device as claimed in claim 1, **characterized in that** the lever mechanism is disposed in the interchangeable skin piercing module.

14. The hand-held device as claimed in claim 1, **characterized in that** the lever mechanism is disposed in a coupling module which can be detachably connected to the handpiece of the electric toothbrush.

15. The hand-held device as claimed in at least one of the preceding claims, insofar as not dependent on claim 10, **characterized in that** the lever mechanism (32) is disposed in the front housing part.

## Revendications

1. Dispositif à main pour piquer localement la peau, comportant
- un boîtier (2) ;
- un dispositif d'entraînement qui est disposé et monté dans le boîtier (2) pour fournir une force d'entraînement ;
- un dispositif de ponction (22) disposé dans le boîtier (2) et comportant au moins une aiguille de ponction (24) disposée sur un porte-aiguille (23) ; et
- un dispositif d'accouplement disposé dans le boîtier (2) et reliant le dispositif d'entraînement au dispositif de ponction (22) de telle sorte que la force d'entraînement puisse être transmise au dispositif de ponction (22) pour déplacer le porte-aiguille (23) avec l'au moins une aiguille de ponction (24) de manière répétée entre une position avant et une position arrière pendant le fonctionnement,
le dispositif d'accouplement présentant un mécanisme à levier (32), qui est conçu pour recevoir une course d'entraînement assurée par le dispositif d'entraînement lors de la transmission de la force d'entraînement côté entraînement par l'intermédiaire d'une entrée du mécanisme à levier (32), pour convertir la course d'entraînement en une course de travail différente de la course d'entraînement, et pour transférer la course de travail au dispositif de ponction (22) par l'intermédiaire d'une sortie du mécanisme à levier (32), le boîtier (2) étant réalisé en plusieurs parties avec des parties de boîtier dissociables les unes des autres, sachant que, avec une partie arrière du boîtier dans laquelle est disposé le dispositif d'entraînement, une poignée et une partie avant du boîtier, il est formé un module de ponction de la peau dans lequel est disposé le dispositif de ponction (22), **caractérisé en ce que** la partie arrière du boîtier est une poignée avec un dispositif d'entraînement d'une brosse à dents électrique.

2. Appareil à main selon la revendication 1, **caractérisé en ce que** le mécanisme à levier (32) est conçu pour convertir la course d'entraînement avec une longueur de course d'entraînement en course de travail avec une longueur de course de travail différente de la longueur de la course d'entraînement.

3. Appareil à main selon la revendication 2, **caractérisé en ce que** la longueur de course de travail est supérieure à la longueur de la course d'entraînement.

4. Appareil à main selon la revendication 2, **caractérisé en ce que** la longueur de la course de travail est inférieure à la longueur de la course d'entraînement.

5. Appareil à main selon au moins l'une des revendications précédentes, **caractérisé en ce que** le mécanisme à levier (32) est réalisé avec au moins deux leviers coopérants (33, 34).

6. Appareil à main selon au moins l'une des revendications précédentes, **caractérisé en ce que** le mécanisme à levier (32) entre l'entrée et la sortie est réalisé sous la forme d'un mécanisme à levier rigide (32).

7. Appareil à main selon au moins l'une des revendications précédentes, **caractérisé en ce que** le porte-aiguille (23) avec l'au moins une aiguille de ponction (24) peut être déplacé lors du passage de la position arrière à la position avant à l'encontre d'une force de rappel fournie par un dispositif de rappel et que la force de rappel est réglée pour fournir au moins une partie d'une force de rappel totale qui doit être appliquée pour le déplacement du porte-aiguille (23) avec l'au moins une aiguille de coupe (24) de la position avant à la position arrière.

8. Appareil à main selon au moins l'une des revendications précédentes, **caractérisé en ce que** dans le boîtier (2), une membrane d'étanchéité sépare l'un de l'autre de manière étanche un espace avant du boîtier d'un espace arrière du boîtier.

9. Appareil à main selon la revendication 8, **caractérisé en ce que** le dispositif de rappel est formé avec la membrane d'étanchéité, de telle sorte que la force de rappel soit établie lorsque des sections de membrane élastiques de la membrane d'étanchéité sont étirées pendant le passage du porte-aiguille (23) avec l'au moins une aiguille de ponction (24) de la position arrière à la position avant.

10. Appareil à main selon la revendication 8 ou 9, **caractérisé en ce que** le mécanisme à levier (32) est disposé dans l'espace arrière du boîtier.

11. Appareil à main selon au moins l'une des revendications précédentes, **caractérisé en ce que** le porte-aiguille (23) du dispositif de ponction (22) est relié directement à la sortie du mécanisme à levier (32).

12. Appareil à main selon la revendication 11, **caractérisé en ce qu'**un élément de sortie, sur lequel est formée la sortie du mécanisme à levier (32), est monté de manière à pouvoir pivoter dans un logement associé sur le porte-aiguille (23) du dispositif de ponction (22).

13. Appareil à main selon la revendication 1, **caractérisé en ce que** le mécanisme à levier est disposé dans le module de ponction de la peau.

14. Appareil à main selon la revendication 1, **caractérisé en ce que** le mécanisme à levier est disposé dans un module d'accouplement qui est connectable de manière dissociable avec le manche de la brosse à dents électrique.

15. Appareil à main selon au moins l'une des revendications précédentes, sauf en cas de référence à la revendication 10, **caractérisé en ce que** le mécanisme à levier (32) est disposé dans la partie avant du boîtier.
